# EUROPÄISCHE PATENTANMELDUNG

(11) **EP 1 593 743 A1**
(43) Veröffentlichungstag der Anmeldung: **09.11.2005**
(21) Anmeldenummer: 04010496.0
(22) Anmeldetag: 03.05.2004
(51) Int. Cl.: C12P 1/04

(54) **Verfahren zur Herstellung von Produkten mit lebenden Bakterien oder Zellen in zweiphasigen Systemen**

(71) Anmelder: Weuster-Botz, Dirk, 85221 Dachau (DE); Pfründer, Holger, 80799 München (DE)
(72) Erfinder: Weuster-Botz, Dirk, 85221 Dachau (DE); Pfründer, Holger, 80799 München (DE)
(74) Vertreter: Vossius & Partner

(57) **Zusammenfassung**

Beschrieben wird ein Verfahren zur Herstellung von einem oder mehreren Produkten in einer Reaktion aus einem oder mehreren Edukten in Gegenwart von lebenden Bakterien oder eukaryontischen Zellen als Biokatalysatoren unter Verwendung eines zweiphasigen Systems, umfassend eine erste biokompatible Phase einer ionischen Flüssigkeit und eine zweite Phase einer wässrigen Flüssigkeit, wobei die ionische Flüssigkeit und die wässrige Flüssigkeit heterogen zueinander sind. Darüber hinaus wird eine Zusammensetzung beschrieben, umfassend ein zweiphasiges System aus einer ersten Phase einer biokompatiblen ionischer Flüssigkeit und einer zweiten Phase einer wässrigen Flüssigkeit, wobei die ionische Flüssigkeit und die wässrige Flüssigkeit heterogen zueinander sind.

## Beschreibung

Die vorliegende Erfindung betrifft ein Verfahren zur Herstellung von einem oder mehreren Produkten in einer Reaktion aus einem oder mehreren Edukten in Gegenwart von lebenden Bakterien oder eukaryontischen Zellen als Biokatalysatoren unter Verwendung eines zweiphasigen Systems, umfassend eine erste biokompatible Phase einer ionischen Flüssigkeit und eine zweite Phase einer wässrigen Flüssigkeit, wobei die ionische Flüssigkeit und die wässrige Flüssigkeit heterogen zueinander sind. Darüber hinaus betrifft die Erfindung eine Zusammensetzung umfassend ein zweiphasiges System aus einer ersten Phase einer biokompatiblen ionischer Flüssigkeit und einer zweiten Phase einer wässrigen Flüssigkeit, wobei die ionische Flüssigkeit und die wässrige Flüssigkeit heterogen zueinander sind.

Verschiedene Dokumente werden im Text dieser Beschreibung zitiert. Der Offenbarungsgehalt der zitierten Dokumente (einschließlich aller Herstellerbeschreibungen, -angaben etc.) ist hiermit per Referenz Teil dieser Beschreibung.

Im Stand der Technik werden verschiedene Ansätze der Herstellung von Feinchemikalien am Beispiel der asymmetrischen Ketonreduktion beschrieben.
Für die asymmetrische Reduktion von Ketonen mittels chemischer Katalyse stehen verschiedene Methoden zur Verfügung. Dazu gehören die boranbasierte Katalyse sowie die direkte Hydrogenierung mit Wasserstoff und die Transferhydrogenierung, die beide von Übergangsmetallkomplexen katalysiert werden (Wills und Hannedouche 2002).
Darüber hinaus wurden für die asymmetrischen Reduktion von Ketonen mit isolierten Enzymen Alkoholdehydrogenasen (ADH) eingesetzt. Diese können entweder aus Wildtyp-Mikroorganismen gewonnen oder rekombinant hergestellt werden (z. B. Hummel et al. 2003). Die Cofaktorversorgung kann dabei entweder durch Zugabe der reduzierten Form (z. B. NADPH) oder durch Zugabe der billigeren oxidierten Form (z. B. NADP) erfolgen. Im zweiten Fall muss die oxidierte Form in die reduzierte Form überführt werden. Dies kann entweder durch die vorhandene ADH erfolgen, die einen zugegebenen Alkohol zum korrespondierenden Keton oxidiert, oder durch ein zweites oxidierendes Enzym (z. B. Oxidation von Ameisensäure durch Formiatdehydrogenase, Seelbach et al. 1996).
Die Enzyme können hierbei entweder in freier Form im Reaktionsmedium vorliegen oder immobilisiert auf Trägern oder in Kapseln.

Als Alternative zu den beschriebenen isolierten Enzyme können auch ganze Zellen eingesetzt werden, die natürlicherweise (Nakamura et al. 2003) oder rekombinant (z. B. Kataoka et al. 2003) eine ADH-Aktivität und ein Regenerationssystem besitzen. Dabei können die Zellen entweder abgetötet, lebendig ruhend oder lebendig wachsend sein. Der erste Fall kann erforderlich sein, wenn zum Beispiel das Substrat die Zellmembran nicht oder nur sehr langsam passieren würde. Dann werden die Zellen beispielsweise mit Detergenzien permeabilisiert. Im zweiten und dritten Fall sind die Zellen intakt. Dadurch sind die Enzyme besser geschützt und der Katalysator ist meistens öfter wieder verwendbar als bei abgetöteten Zellen. Der Metabolismus ruhender Zellen ist nicht aktiv. Wachsenden Zellen stehen Nährstoffe und eine Energiequelle zur Verfügung, sie sind metabolisch aktiv.
Wie bei isolierten Enzymen können auch die Zellen in freier oder immobilisierter Form vorliegen.

Für die Ketonreduktionen ist allgemein bekannt, dass die Substrate und Produkte oft schlecht wasserlöslich sind. Zusätzlich haben diese Substanzen in höheren Konzentrationen oft einen inhibierenden oder toxischen Effekt auf den Biokatalysator. Um die Substratverfügbarkeit zu erhöhen und höhere Produktausbeuten zu erreichen, ohne die inhibierenden Konzentrationen zu überschreiten kann eine Lösungsmittelphase in den Prozess eingebracht werden. Dies ist bisher üblicherweise ein leicht verfügbares organisches Lösungsmittel.

Zu beachten ist bei entsprechendem Vorgehen unter anderem, dass das verwendete Lösungsmittel selbst keinen negativen Einfluss auf den Biokatalysator haben soll.
Für ganze Zellen wurde eine Reihe von Kriterien entwickelt, die die Eignung von Lösungsmitteln abschätzen. Das gebräuchlichste Kriterium ist der log P-Wert, der Logarithmus des Verteilungskoeffizienten des Lösungsmittels zwischen Wasser und n-Oktanol (Laane et al. 1987).
Für isolierte Enzyme scheint jedoch die Funktionalität des Lösungsmittels ein besseres Kriterium als der log P-Wert zu sein (Villela et al. 2003). Zudem besteht die Möglichkeit gezielt nach lösungsmitteltoleranten Enzymen und Organismen zu suchen oder Enzyme durch verschiedene Ansätze wie "directed evolution" oder "rational design" in diese Richtung zu verändern.

In alternativen Zweiphasensystemen bilden superkritische Fluide oder ionische Flüssigkeiten die Lösungsmittelphase. Für die Enzymkatalyse sind diese Verfahren patentiert oder Gegenstand einer Patentanmeldung (US Patent No 4,925790; WO 02/038784). Für die Ganzzellkatalyse sind mittlerweile vereinzelt erste Ansätze mit überkritischen Fluiden (Matsuda et al. 2003) und ionischen Flüssigkeiten (Cull et al. 2001, Howarth et al. 2001) beschrieben worden. Cull et al. führten Biotransformationen in Gegenwart der ionischen Flüssigkeit BMIM PF₆ (20 Volumenprozent) durch. Das Substrat wurde in der ionischen Flüssigkeit vorgelegt. Ein Einsatz ionischer Flüssigkeiten als Extraktionsmittel wurde jedoch bisher nicht beschrieben. Für diese Reaktion wird kein Cofaktor benötigt. Howarth et al. führten ebenfalls Biotransformationen in BMIM PF₆ (etwa 91 Volumenprozent) durch. Die ionische Flüssigkeit wurde als Lösungsmittel eingesetzt. Das Wasser wurde lediglich zugegeben, um eine bei organischen Lösungsmitteln beobachtete Inaktivierung der Enzyme zu vermeiden. Das NADPH-Regenerationssystem der Zellen wurde nicht genutzt.

In der chemischen Katalyse wurden in der Vergangenheit vor allem Prozesse mit Übergangsmetall katalysierten Transferhydrogenierung entwickelt (Wills und Hannedouche 2002, Rautenstrauch et al. 2003, Himeda et al. 2003). Gründe dafür sind die zum Teil hohen Aktivitäten der Katalysatoren und die hohen erzielten Produktreinheiten. So konnten Produkte mit 99 % ee und 100 % Ausbeute gewonnen werden (Ohkuma et al. 2002). In den meisten Fällen aber mußten die Produkte nach der Synthese noch in mehreren Schritten aufgereinigt werden (z. B. durch Umkristallisierung), um einen hohen Enantiomerenüberschuss zu erhalten (Wills und Hannedouche 2002).
Aufgrund dieser notwendigen, z.T. extrem aufwändigen Schritten erscheint damit für viele interessante Produkte die biokatalytische Synthese geeigneter als die chemische Katalyse (z. B. Hage et al 2001). Neben den ökonomischen Vorteilen bietet die so genannte weiße Biotechnologie zudem viele ökologische Vorteile.
Ein Beispiel für ein Verfahren der weißen Biotechnologie ist die enzymatisch katalysierte Synthese chiraler Alkohole. Die Kosten für Enzyme und Cofaktoren verhindern jedoch oft einen effizienten Prozess mit isolierten Enzymen. Bei der Verwendung ganzer Zellen entfällt die aufwändige Gewinnung der isolierten Enzyme. Außerdem zeigen viele isolierte Enzyme beim Einsatz von Zweiphasensystemen eine reduzierte Stabilität an der Flüssig-Flüssig-Grenzfläche, der durch weitere mehr oder weniger aufwändige Maßnahmen entgegen gewirkt werden muss (Krishna 2002).
Alternativ wurde auch die Ganzzellkatalysierte Synthese chiraler Alkohole beschrieben. Wie bei der Katalyse mit isolierten Enzymen wird bei der Ganzzellbiotransformation die Effizienz des Prozesses oft durch die schlechte Wasserlöslichkeit der Substrate und Produkte sowie deren inhibierende oder toxische Wirkung auf den Biokatalysator beeinträchtigt.
Für die Biokatalyse in Zweiphasensystemen mit organischen Lösungsmitteln wurde beschrieben, dass sowohl die erzielbare Produktkonzentration als auch die volumetrische Produktivität durch die Verwendung einer Lösungsmittelphase gesteigert werden können. Allerdings wird sehr oft ein inhibierender oder toxischer Einfluss von organischen Lösungsmitteln auf den Biokatalysator beobachtet.
Hinzu kommt, dass die leichte Flüchtigkeit der organischen Lösungsmittel zu Problemen bei der Wiedergewinnung, Handhabung und Sicherheit und zu Umweltbelastung führt.
Die Verwendung altemativer Zweiphasensysteme für die Ganzzellbiokatalyse führte bisher im Gegensatz zur Enzymkatalyse nicht zu effizienten Prozessen.
Mit überkritischem CO₂ als Lösungsmittel für die asymmetrische Reduktion von 2-Fluoracetophenon mit immobilisierten *Geotrichum candidum-*Zellen wird eine Ausbeute von 8 % bei einer Raumzeitausbeute von 0,2 g*L⁻¹*h⁻¹ erreicht (Matsuda et al. 2003).
Bei der Biotransformation von 1,3 dicyanobenzene mit *Rodococcus* R312-Zellen in einem Zweiphasensystem mit 20 % BMIM-PF₆ wurde nur 1 g L⁻¹ Substrat eingesetzt. Nach der Reaktion wurden circa 0,6 mmol L⁻¹ Produkt in der wässrigen Phase nachgewiesen. Ein Einsatz der ionischen Flüssigkeit als Extraktionsmittel wurde in diesem Zusammenhang bisher weder beschrieben noch vorgeschlagen. Für die von Cull et al. 2000 beschriebene Reaktion wird darüber hinaus kein Cofaktor benötigt.
Die Reduktion verschiedener Ketone mit immobilisierten Bäckerhefe-Zellen in 91 % BMIM-PF₆ führte zwar zu Ausbeuten von 22 bis 75 % bei circa 100 mmol*L⁻¹ eingesetztem Substrat (Howarth et al. 2001). Die Reinheit betrug jedoch lediglich 76 bis 95 % ee, so dass aufwendige, zusätzliche Reinigungsschritte nötig sind, um die Feinchemikalien in notwendiger Qualität zu erhalten. Bei keiner der untersuchten Reduktionen konnte durch den Einsatz einer ionischen Flüssigkeit eine entscheidende Verbesserung in Ausbeute und Reinheit zu einem alternativen Verfahren erzielt werden. Ferner wurde bei diesem Prozess das Regenerationssystem der Zellen nicht genutzt. Folglich konnte die Reaktion nur so lange fortschreiten, bis die anfänglich in den Zellen vorhandene Menge NADPH verbraucht war. Dies verhindert zusätzlich eine effiziente Prozessführung.

Aufgabe der vorliegenden Erfindung war somit ein Verfahren bereitzustellen, das eine effektive und kostengünstige Herstellung von Feinchemikalien ermöglicht, welches eine aufwendige Reinigung der hergestellten Produkte nach Abschluss der Synthese/Biokatalyse nicht mehr notwendig macht.

Diese Aufgabe wird durch die Bereitstellung der in den Ansprüchen gekennzeichneten Ausführungsformen gelöst.

Folglich betrifft die vorliegende Erfindung ein Verfahren zur Herstellung von einem oder mehreren Produkten in einer Reaktion aus einem oder mehreren Edukten in Gegenwart von lebenden Bakterien oder eukaryontischen Zellen als Biokatalysatoren unter Verwendung eines mindestens zwei flüssige Phasen umfassenden Systems, umfassend eine erste biokompatible Phase einer ionischen Flüssigkeit und eine zweite Phase einer wässrigen Flüssigkeit, wobei die ionische Flüssigkeit und die wässrige Flüssigkeit heterogen zueinander sind und im Verfahren
(a) die erste Phase als Eduktreservoir dient; und
(b) die zweite Phase die lebenden Bakterien oder eukaryontischer Zellen enthält;
wobei die Phasen intensiv miteinander in Kontakt gebracht werden, das eine oder die mehreren Edukte durch Stofftransportvorgänge in die zweite Phase eintreten, dort durch die Biokatalysatoren in das eine oder die mehreren Produkte umgesetzt werden und das eine oder die mehreren Produkte nach der Umsetzung aus dem zweiphasigen System abgetrennt werden.
Produkte des erfindungsgemäßen Herstellungsverfahren sind vorzugsweise Feinchemikalien
Der Begriff "Feinchemikalien" ist ein Begriff zur Bezeichnung von Chemikalien unterschiedlicher Reinheitsgrade, die im Maßstab kleiner 10.000 Jahrestonnen hergestellt werden.
Diese Feinchemikalien werden aus Edukten durch Enzyme in den lebenden bakteriellen oder eukaryontischen Biokatalysatoren zu den gewünschten Produkten umgesetzt.
Die Gruppe der Bakterien umfasst im Zusammenhang mit dieser Erfindung sowohl Eubakterien, als auch Archaebakterien. Der Begriff "Bakterien" bezeichnet somit sämtliche prokaryontischen Organismen.
Eine Mindestvoraussetzung für als lebend geltende Bakterien und eukaryontische Zellen im Zusammenhang mit der Erfindung ist die Aufrechterhaltung der Integrität der Bakterien/Zellmembran. Mögliche Verfahren zur Bestimmung der Integrität der Bakterien/Zellmembran sind in den angefügten Beispielen beschrieben. Vorzugsweise werden Organismen als lebende Bakterien und eukaryontische Zellen bezeichnet, wenn zumindest Teile des Organismus eigenen Enzym-Apparates funktionell sind. Dies betrifft insbesondere Enzyme, Enzymkaskaden, Stoffwechselabschnitte und/oder ganze Stoffwechselnetzwerke, soweit diese an der Umsetzung des/der Edukts/e in das/die Produkt(e) beteiligt sind. Insbesondere sind hierbei auch enzymatische Regenerationssysteme, wie Regenerationssysteme von Cofaktoren umfasst.

Das mindestens zwei flüssige Phasen umfassende System des erfindungsgemäßen Verfahrens ist dadurch gekennzeichnet, dass die erste biokompatible Phase einer ionischen Flüssigkeit und die zweite Phase einer wässrigen Flüssigkeit heterogen zueinander sind. Werden diese beiden Phasen miteinander vermischt, so entsteht eine Emulsion. Dies bedeutet, dass die Phasen bei Durchmischung des Systems erhalten bleiben und keine Mischung sondern lediglich ein Gemisch zweier Flüssigkeiten entsteht. Vorzugsweise ist das Phasensystem ein System, das zwei flüssige Phasen umfasst, wobei diese zwei flüssigen Phasen die oben beschriebene erste biokompatible Phase einer ionischen Flüssigkeit und zweite Phase einer wässrigen Flüssigkeit sind. Durch das erfindungsgemäßen Verfahren sind explizit auch Verfahren umfasst, die weitere Phasen, insbesondere feste Phasen oder Gasphasen umfassen.

Die Definition des bevorzugten zweiphasigen Systems im Zusammenhang mit dem erfindungsgemäßen Verfahren bezieht sich nur auf die Definition der flüssigen Phasen im System. Entsprechend sind durch das bevorzugte erfindungsgemäßen Verfahren auch Verfahren umfasst, die zusätzlich zu den zwei flüssigen Phasen weitere Phasen, wie feste Phasen oder Gasphasen umfassen.

Ionische Flüssigkeiten, die für das erfindungsgemäße Verfahren geeignet sind, sind dadurch gekennzeichnet, dass sie heterogen zu wässrigen Flüssigkeiten sind. Darüber hinaus ist die Biokompatibilität der ionischen Flüssigkeit ein essentielles Merkmal. Dies bedeutet, dass die ionische Flüssigkeit die Funktionalität/Integrität der Membranen der Bakterien/Zellen nicht negativ beeinträchtigt. Eine funktionale/integre Membran ist im Zusammenhang mit dieser Erfindung dadurch gekennzeichnet, dass Konzentrationsgradienten zwischen der Membraninnenseite und der Membranaußenseite bestehen und aufrechterhalten werden. Entsprechend gilt die Funktionalität/Integrität der Membranen der Bakterien/Zellen als nicht negativ beeinträchtigt, sofern ein Konzentrationsgradient zwischen der Membraninnenseite und der Membranaußenseite aufrecht erhalten bleibt. Verfahren zur Testung der Funktionalität/Integrität von Membranen in Organismen sind dem Fachmann bekannt und z.B. beschrieben in Beispiel 3.
Durch die Auswahl der Anionen und Kationen der ionischen Flüssigkeiten können deren Eigenschaften über weite Bereiche eingestellt werden. Damit ist es möglich, dass für jeden Mikroorganismus bzw. Zelltyp und jede Kombination von chemischen und physikalischen Reaktionsbedingungen ein geeignetes Lösungsmittel ausgewählt wird. Geeignete Verfahren zur Analyse der Eignung einer ionischen Flüssigkeit für einen Mikroorganismus bzw. Zelltyp sind in den angehängten Beispielen beschrieben.
Die Eigenschaft der ersten Phase als Eduktreservoir zu dienen (und ggf. auch als Produktreservoir) ermöglicht die Verwendung (und Herstellung) von Stoffen, die in wässrigen Phasen (d.h. der zweiten Phase) nur eine geringe Stabilität besitzen.
Wässrige Flüssigkeiten, die für das erfindungsgemäße Verfahren geeignet sind, sind dadurch gekennzeichnet, dass Wasser als Lösungsmittel verwendet wird. Vorzugsweise sind gelöste Bestandteile enthalten, welche die Aufrechterhaltung der für den jeweiligen Biokatalysator spezifischen physiologischen Bedingungen ermöglichen. Diese physiologischen Bedingungen umfassen beispielsweise eine bestimmte lonenstärke, einen bestimmten pH-Wert oder -Bereich, eine bestimmte Temperatur oder einen bestimmten Druck.
Im Zusammenhang mit der Erfindung definiert der Begriff "Stofftransportvorgänge" Mechanismen, die den Stofftransport von der abgebenden in die aufnehmende Phase ermöglichen. Im einzelnen können dies sein:
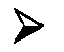 konvektiver Stofftransport in der Kernströmung der abgebenden Phase,
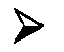 diffusiver Stofftransport von der Kernströmung der abgebenden Phase durch die Phasengrenzschicht zur Phasengrenze,
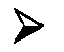 Stoffdurchgang an der Phasengrenze (in der Regel thermodynamisches Gleichgewicht),
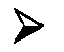 diffusiver Stofftransport von der Phasengrenze durch die Phasengrenzschicht zur Kernströmung der aufnehmenden Phase und
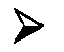 konvektiver Stofftransport in der Kernströmung der aufnehmenden Phase.

Gemäß des erfindungsgemäßen Verfahrens werden die zwei Phasen intensiv miteinander in Kontakt gebracht. Dieses in Kontaktbringen kann durch eine Durchmischung der Flüssigkeiten erreicht werden. Hierbei wird durch einen hohen Leistungseintrag eine Dispergierung von Phase 1 in Phase 2 oder Phase 2 in Phase 1 erreicht. Ziel ist eine große Stoffaustauschfläche und hohe Differenzgeschwindigkeit zwischen den Phasen, um möglichst dünne Grenzschichten zu erreichen. Beispiele für Verfahrensschritte zur Durchmischung sind das Schütteln oder Rühren des Reaktionsansatzes mit dem Zweiphasensystem.
Die Abtrennung der Produkte aus dem zweiphasigen System kann durch beliebige, dem Fachmann bekannte Abtrennungsverfahren erreicht werden. Beispiele für Verfahren zur Abtrennung der Produkte sind hierin u.a. in Beispiel 5 beschrieben.

Durch die Bereitstellung des erfindungsgemäßen Verfahrens wurden überraschenderweise effizientere Prozesse zur Ganzzell-katalysierten Herstellung von Feinchemikalien entwickelt. Neben einer Steigerung der Ausbeute, der Produktkonzentration und der Raumzeitausbeute wird durch dieses Verfahren auch die Produktreinheit erhöht.
Die Verwendung von zu Wasser heterogenen ionischen Flüssigkeiten nutzt die Vorteile eines Zweiphasensystems, die niedrige Konzentration von potenziell zellschädigenden Substraten und Produkten in der Katalysatorphase und die hohen Konzentrationen dieser Stoffe in der Lösungsmittelphase, die für eine gute Prozessausbeute nötig sind. Durch die unerwarteten zellschonenden Eigenschaften der ionischen Flüssigkeiten können diese Vorteile in vollem Umfang genutzt werden; siehe Beispiel 5.
Die zellschonenden Eigenschaften gewährleisten eine Aufrechterhaltung der Integrität der Bakterien/Zellmembran, die Voraussetzung für eine Umsetzung ist, an der
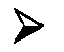 mehr als ein Enzym beteiligt ist und/oder
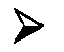 Stoffwechselabschnitte beteiligt sind und/oder
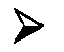 Ganze Stoffwechseinetzwerke beteiligt sind und/oder
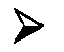 (Transport-)Metabolite beteiligt sind.

In den angefügten Beispielen werden u.a. Redoxreaktionen beschrieben, bei denen neben einer bakterieneigenen Alkoholdehydrogenase (ADH) für die Stoffumsetzung noch weitere bakterieneigene Transportmetabolite für den Elektronentransport (als Hydridion) und Teile des bakterieneigenen Zentralstoffwechsels (Pentosephosphatweg) des Biokatalysators zur Regeneration der Transportmetabolite (Cofaktoren) benötigt werden.

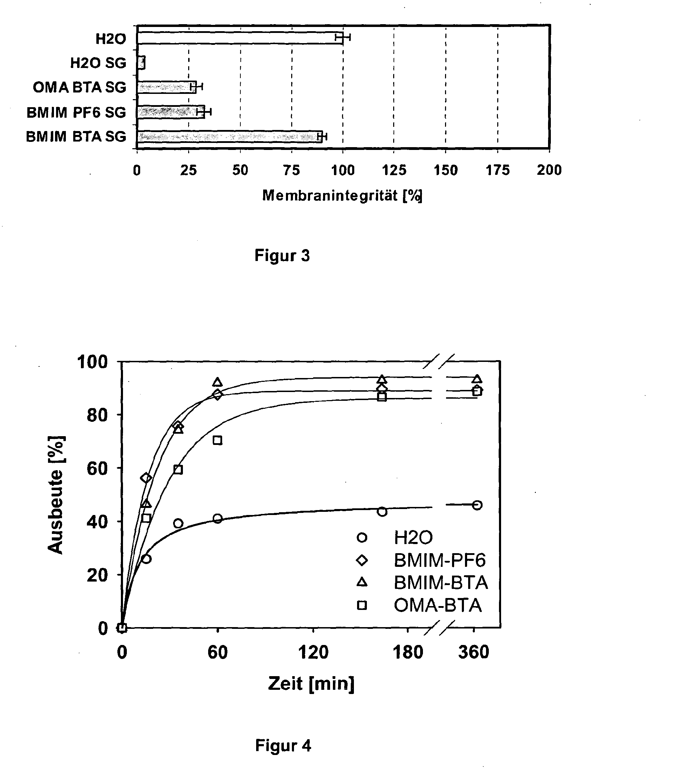

Aus dem Stand der Technik war bekannt, dass viele organische Lösungsmittel sehr schädlich für die Zellmembran sind. Die zellschädigenden Eigenschaften organische Lösungsmittel werden deren Fähigkeit sich in die Membran einzulagern zugeschrieben. Decan, das mit 5,6 einen hohen log P-Wert besitzt, zeigt eine geringere Zellschädigung, wie aus vergleichbaren Literaturdaten zu erwarten war (Inoue A und Horikoshi K, 1991). Es ist jedoch als Lösungsmittel aufgrund des unpolaren Charakters schlecht geeignet. Die mit Wasser vollständig mischbaren ionischen Flüssigkeiten 1,3-Dimethylimidazolium Diphosphat (MMIM DiPO₄), 1,3-Butyl-Methylimidazolium Octylsulphat (BMIM-OcSO₄) und 1,3-Butyl-Methylimidazolium Tetrafluoroborat (BMIM BF₄) führen ebenso zu einer mehr oder weniger ausgeprägten Zellschädigung.
Die nicht mit Wasser mischbaren (bei Vermischung Emulsionen bildenden) ionischen Flüssigkeiten BMIM PF₆, BMIM BTA und OMA BTA zeigen überraschenderweise keine Beeinträchtigung der Membranintegrität. Es ist im Gegenteil sogar eine Verstärkung der Barrierefunktion zu beobachten. Wie in Figur 3 dargestellt, wird diese unter Reaktionsbedingungen durch das Substrat und das Produkt wieder aufgehoben, sodass es nicht zu einer im Vergleich zum rein wässrigen Ansatz erhöhten Stofftransportlimitierung kommt (siehe Beispiel 5).
Ein überraschender Vorteil, der sich aus der guten Biokompatibilität der ionischen Flüssigkeiten ergibt, ist die Möglichkeit der Nutzung der Regenerationssysteme des Biokatalysators von Reaktionspartnern. Die Gruppe der zu regenerierenden Reaktionspartner umfasst insbesondere Cofaktoren, d.h. kleinere Moleküle, die im Biokatalysator vorhanden sind und für die katalytische Funktion des Katalysators notwendig sind.
Bei Vorlage des Cosubstrats in der wässrigen Phase kann der Zusatz teurer Cofaktoren vermieden werden, da die intakten Zellen diesen immer wieder regenerieren können und aufgrund der intakten Membran nicht verlieren. Dies bedeutet unendliche Zykluszahlen (Menge des gebildeten Produkts pro Menge zugesetzten Cofaktors).
Durch das erfindungsgemäße Verfahren wird gewährleistet, dass durch die Verwendung von zu Wasser heterogenen ionischen Flüssigkeiten eine sehr hohe Produktreinheit erreicht werden kann. Der Enantiomerenüberschuss von (R)-1-(4-Chlorphenyl)ethanol wurde im zweiphasigen System ionische Flüssigkeit/Wasser im Vergleich zum rein wässrigen Ansatz von 98,0 % auf über 99,5 % gesteigert (siehe Beispiel 5).
Durch die räumliche Trennung des Biokatalysators und des Produkts in zwei verschiedenen Phasen sowie die Vermeidung von Zellaggregationen, die bei Verwendung von organischen Lösungsmitteln im Stand der Technik oft beobachtet wurde (Cull et al. 2000), wird eine einfache Produktaufbereitung ermöglicht. Die Lösungsmittelphase kann z.B. durch einfache Sedimentation oder Zentrifugation gewonnen werden. Das Produkt kann daraus durch Destillation, Extraktion, Pervaporation (WO 03/013685) Membranfiltration (WO 03/039719) oder ein anderes Standardverfahren gewonnen werden. Außerdem können die Zellen für einen neuen Ansatz wieder verwendet werden.
Die ionische Flüssigkeit kann nach der Produktabtrennung ebenfalls wieder verwendet werden. Im Gegensatz zur Arbeit mit organischen Lösungsmitteln ist dies durch den extrem geringen Dampfdruck verlustfrei möglich. Gegebenenfalls kann die ionische Flüssigkeit auch leicht gereinigt werden.
Ferner stellt die gefahrlose Handhabung ionischer Flüssigkeiten gegenüber organischen Lösungsmitteln einen großen Vorteil dar. Die Arbeitssicherheit wird erhöht, die Umweltbelastung reduziert.

Wie bereits oben beschrieben können Bakterien/Zellen verwendet werden, die die nötige Aktivität zur Regeneration von Reaktionspartnem wie Cofaktoren natürlicherweise oder rekombinant besitzen. Entsprechend betrifft eine Ausführungsform der Erfindung ein Verfahren, bei welchem die Reaktion unter Ausnutzung der bakerieneigenen/zelleigenen Regeneration von verbrauchten Reaktionspartnem abläuft. "Bakerieneigen/Zelleingenen" definiert in diesem Zusammenhang sowohl Regenerationssysteme, die natürlich in den verwendeten Bakterien/Zellen vorkommen, als auch transgene Regenerationssysteme.
Hierbei ist jedes bekannte Regenerationssystem für Reaktionspartner, wie Cofaktoren, bei der Katalyse oben beschriebener Umsetzungsreaktionen umfasst. Beispiele für Cofaktoren und deren Regenerationssysteme umfassen u.a.:
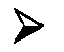 Nicotinamidadenindinucleotid (NAD(H)): Übertragung von Hydridionen
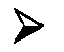 Nicotinamidadenindinucleotidphosphat (NADP(H)): Übertragung von Hydridionen
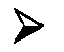 Flavinadenindinucleotid (FAD): Übertragung von Elektronen
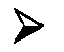 Thiaminpyrophosphat: Übertragung von Aldehyden
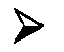 Pyridoxalphosphat: Übertragung von Aminogruppen
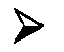 Biocytin: Übertragung von CO2
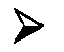 Tetrahydrofolat: Übertragung von C1-Gruppen
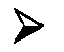 Coenzym A: Übertragung von Acylgruppen
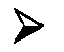 5'-Desoxyadenosylcobalamin: Übertragung von H-Atomen und Alkylgruppen

In einer bevorzugten Ausführungsform ist das erfindungsgemäße Verfahren dadurch gekennzeichnet, dass die ionische Flüssigkeit der ersten Phase in einem Temperaturbereich von 0°C bis 90°C flüssig ist.

In einer ebenfalls bevorzugten Ausführungsform treten das eine oder die mehreren Produkte durch Stofftransportvorgänge in die erste Phase über und diese erste Phase dient zusätzlich als in-situ-Extraktionsmittel. Diese Ausführungsform ermöglicht eine zusätzlich vereinfachte Abtrennung der hergestellten Produkte. Geeignete in-situ-Extraktionsmittel sind dadurch gekennzeichnet dass, diese nicht mischbar mit Wasser sind, unter Reaktionsbedingungen (0-90 °C) flüssig sind, ausreichend hohe Verteilungskoeffizienten für Produkt(e) besitzen (z.B. größer 10) und durch Biokompatibilität (=keine Beeinträchtigung der Funktionalität der Zellmembran oder Membranintegrität).

Vorzugsweise ist der Stofftransportvorgang, durch den das eine oder die mehreren Edukte in die zweite Phase eintreten und/oder das eine oder die mehreren Produkte in die erste Phase eintreten, ausgewählt aus der Gruppe bestehend aus konvektivem Stofftransport, diffusivem Stofftransport und Stoffdurchgang an der Phasengrenze.

lonische Flüssigkeiten des erfindungsgemäßen Verfahrens sind vorzugsweise ausgewählt ist aus Hexafluorophosphatsalzen (PF₆⁻) und Bis-Trifluormethansulfonamidsalzen (BTA⁻). Besonders bevorzugte Beispiele umfassen 1,3-Butyl-Methylimidazolium Hexafluorophosphat (BMIM PF₆), 1,3-Butyl-Methylimidazolium Bis-Trifluormethansulfonamid (BMIM BTA) und Trioctylammonium Bis-Trifluormethansulfonamid (OMA BTA).

Vorzugsweise umfasst die zweite Phase einen Puffer zur Aufrechterhaltung eines pH-Wertes in dieser Phase. Geeignet sind in diesem Zusammenhang alle als mit Bakterien und eukaryontischen Zellen als biokompatibel bekannten Puffersalze. Vorzugsweise sind die Puffersalze ausgewählt aus der Gruppe bestehend aus Salzen der Citronensäure, Salzen der Essigsäure, Histidin, Salzen der Phosphoglycerinsäure, Hydrogencarbonatsalzen, Maleatsalzen, Dihydrogenphosphatsalzen, Imidazol, Triethanolamin, Glycylglycin, Tris-(hydroxymethyl)-aminomethan und Pyrophosphatsalze.
Die zweite Phase ist vorzugsweise eine wässrige Nährlösung für die lebenden Bakterien oder eukaryontischer Zellen. Grundsätzlich sind alle bekannten Nährlösungen geeignet. Bevorzugt sind synthetische wässrige Nährlösungen. Hierbei handelt es sich um Nährlösungen, die aus einzelnen chemisch definierten Stoffen zusammengesetzt sind und keine undefinierten Komplexbestandteile wie beispielsweise verschiedene Tier-, Mikroorganismen- oder Pflanzenextrakte enthalten. Besonders bevorzugt sind Nährsalzlösungen, da sich lonen nicht in ionischen Flüssigkeiten lösen können. Die bedeutet, dass Produkte nach Trennung von der ionischen Flüssigkeit und der wässrigen Phase bereits sehr sauber gewonnen werden können. Besonders bevorzugt sind hierbei Nährsalzlösungen, die mindestens eines der folgenden Nährsalze umfassen: Phosphatsalze, Sulfatsalze, Chloridsalze und Carbonatsalze, die als Kationen bevorzugt Natrium, Kalium, Magnesium, Calcium, Mangan, Eisen, Kobalt, Nickel, Borat, Kupfer und/oder Zink enthalten.

Für das erfindungsgemäße Verfahren sind grundsätzlich alle Mikroorganismen und Zellen geeignet, die eine gewünschte Biotransformationsaktivität besitzen. Da heute gewünschte Enzymaktivitäten in nahezu beliebigen Wirtssystemen exprimierbar sind, sind für das erfindungsgemäße Verfahren sowohl Wildtypzellen, als auch rekombinante Wirtssysteme geeignet.
Wildtyp-Organismen umfassen u.a. Lactobacillus spec., Rhodococcus spec., Synechococcus spec., Bacillus spec., Candida spec., Caldariomyces spec., Pseudomonas spec., Gluconobacter spec., Cellulomonas spec., Clostridium spec., Agaricus spec., Coriolus spec., Brevibacterium spec., Trichoderma spec., Streptomyces spec., Rhodotorula spec., Aspergillus spec., Staphylococcus spec., Thermoanaerobicum spec.
Als Wirtssysteme sind bevorzugt Escherichia coli, Saccharomyces, insbesondere cerevisiae, Pichia pastoris, Bacillus subtilis, Pseudomonas, insbesondere thuringiensis, Hansenula polymorpha und Aspergillus, insbesondere niger..
Ebenso sind als Wirtssystem auch Pflanzenzellen z.B. aus Arabidopsis und Tabak und Insektenzellen geeignet.
Geeignete Säugerzellen umfassen u.a. HEK-Zellen, HeLa-Zellen, BHK-Zellen, CHO-Zellen und Hybridoma-Zellen.
Darüber hinaus gelten phototrophe Mikroorganismen wie beispielsweise Cyanobakterien und Mikroalgen und Protozoen wie beispielsweise Tetrahymena spec., Euglena spec. oder Cryptothecodinium spec. als geeignete Organismen im Zusammenhang mit der vorliegenden Erfindung.

Vorzugsweise wird das erfindungsgemäße Verfahren in einem Reaktor ausgeführt, der ein Rührkessel ist.
Generell sind Reaktionssysteme geeignet, mit denen
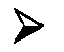 eine Dispergierung von Phase 1 (disperse Phase) in Phase 2 (kontinuierliche Phase) oder eine Dispergierung von Phase 2 (disperse Phase) in Phase 1 (kontinuierliche Phase) erfolgen kann und
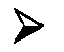 die beiden heterogenen Flüssigkeitsphasen nach Abbruch der Reaktion voneinander getrennt werden können (entweder im Schwerefeld oder im Zentrifugalfeld).
Beispiele für entsprechende alternative Reaktoren zur Rührkesseln umfassen z.B. Blasensäulen und Strömungsrohre mit statischen Mischem.
In einer bevorzugten Ausführungsform ist das erfindungsgemäße Verfahren ein Satz-Verfahren (batch-Verfahren), bei dem das eine oder die mehreren Produkte nach Abschluss der Umsetzung aus dem zweiphasigen System abgetrennt wird/werden. Hierbei ist es möglich die Vorteile der ionischen Flüssigkeit als in-situ Eduktreservoir und in-situ Extraktionsmittel am effektivsten zu nutzen.
Altemativ kann das Verfahren auch als kontinuierliches oder halbkontinuierliches Verfahren ausgeführt werden.

Vorzugsweise wird das erfindungsgemäße Verfahren so durchgeführt, dass die Temperatur der zweiten Phase während der Umsetzung in einem Bereich von 0°C bis 90°C liegt. Vorzugsweise wird das erfindungsgemäße Verfahren so durchgeführt, dass der pH-Wert in der zweiten Phase während der Umsetzung in einem Bereich von 2 bis 10 liegt.

In einer bevorzugten Ausführungsform ist das erfindungsgemäße Verfahren dadurch gekennzeichnet, dass die Edukte in der wässrigen Phase toxisch für die Bakterien oder eukaryontischen Zellen sind.
Die Definition der Toxizität bezieht sich hierbei auf die Konzentration, die üblicherweise notwendig ist, um eine Umsetzung der Edukte zu dem gewünschten Produkt zu ermöglichen. Die im Stand der Technik genannten Konzentration der Edukte für eine effektive Umsetzung gelten im allgemeinen als toxisch für Bakterien oder eukaryontische Zellen (führen zum Verlust der Membranintegrität). Durch das hier beschriebenen Verfahren ist es möglich, die Konzentration der Edukte (und/oder Produkte) in einem Konzentrationsbereich zu gewährleisten, der die Membranintegrität der Bakterien/Zellen nicht soweit schädigt, dass diese sterben.

In einer weiteren Ausführungsform umfasst die Erfindung eine Zusammensetzung umfassend ein zweiphasiges System aus einer ersten Phase einer biokompatiblen ionischer Flüssigkeit und einer zweiten Phase einer wässrigen Flüssigkeit, wobei die ionische Flüssigkeit und die wässrige Flüssigkeit heterogen zueinander sind, wobei
(a) die erste Phase Edukte für eine Umsetzung zu Produkten durch einen Biokatalysator enthält; und
(b) die zweite Phase die lebenden Bakterien oder eukaryontischer Zellen als Biokatalysator enthält.

Vorzugsweise ist die Zusammensetzung dadurch gekennzeichnet, dass die erste Phase zusätzlich als in-situ-Extraktionsmittel geeignet ist. Bevorzugt ist die ionische Flüssigkeit der ersten Phase eine der oben näher definierten ionischen Flüssigkeiten.
Vorzugsweise umfasst die zweite, wässrige Phase einen Puffer zur Aufrechterhaltung eines pH-Wertes in dieser Phase. Geeignete Puffersalze sind im Zusammenhang mit dem erfindungsgemäßen Verfahren oben beschrieben.
Darüber hinaus bevorzugt ist die zweite Phase eine oben beschriebene Nährlösung für die lebenden Bakterien oder eukaryontischer Zellen
Die in der erfindungsgemäßen Zusammensetzung enthaltenen Bakterien oder eukaryontischen Zellen, die als Biokatalysator dienen, sind vorzugsweise ausgewählt aus den oben beschriebenen Bakterien oder eukaryontischen Zellen.

In einer besonders bevorzugten Ausführungsform der erfindungsgemäßen Zusammensetzung sind die Edukte in der wässrigen Phase toxisch für die Bakterien oder eukaryontischen Zellen.

### Figur 1:

Verlauf der Reduktion von 4-Chloracetophenon zu (R)-1-(4-Chlorphenyl)ethanol mit Lactobacillus kefir in 20 % BMIM BTA. Dargestellt sind die Konzentrationen in BMIM BTA.

### Figur 2:

Verteilungskoeffizienten von 4-Chloracetophenon und 1-(4-Chlorphenyl)ethanol zwischen verschiedenen ionischen Flüssigkeiten (IL) und Wasser im Vergleich zu einem guten organischen Lösungsmittel (MTBE) und Wasser.

### Figur 3:

Vergleich der Membranintegrität von *L. kefir* unter Reaktionsbedingungen mit Substrat (S) und Glukose (G) in 20 % ionischer Flüssigkeit im Vergleich zum rein wässrigen Ansatz mit und ohne Substrat.

### Figur 4:

Verlauf der asymmetrischen Reduktion von 4-Chloracetophenon mit *Lactobacillus kefir* in 20 % BMIM-BTA, BMIM PF₆ und OMA BTA im Vergleich zum rein wässrigen Ansatz.

### Figur 5:

Vergleich der Membranintegrität von *L*. *kefir* unter Reaktions-bedingungen jedoch ohne Substrat in 20 % ionischer Flüssigkeit oder organischen Lösungsmitteln im Vergleich zum rein wässrigen Ansatz.

### Figur 6:

Vergleich des Enantiomerenüberschusses (ee) von (R)-1-(4-Chlorphenyl)-ethanol bei Verwendung ionischer Flüssigkeiten im Vergleich zum rein wässrigen Ansatz.

Die Beispiele erläutern die Erfindung.

### Beispiel 1:

### Ganzzellbiotransformation im zweiphasigen System ionische Flüssigkeit/Wasser

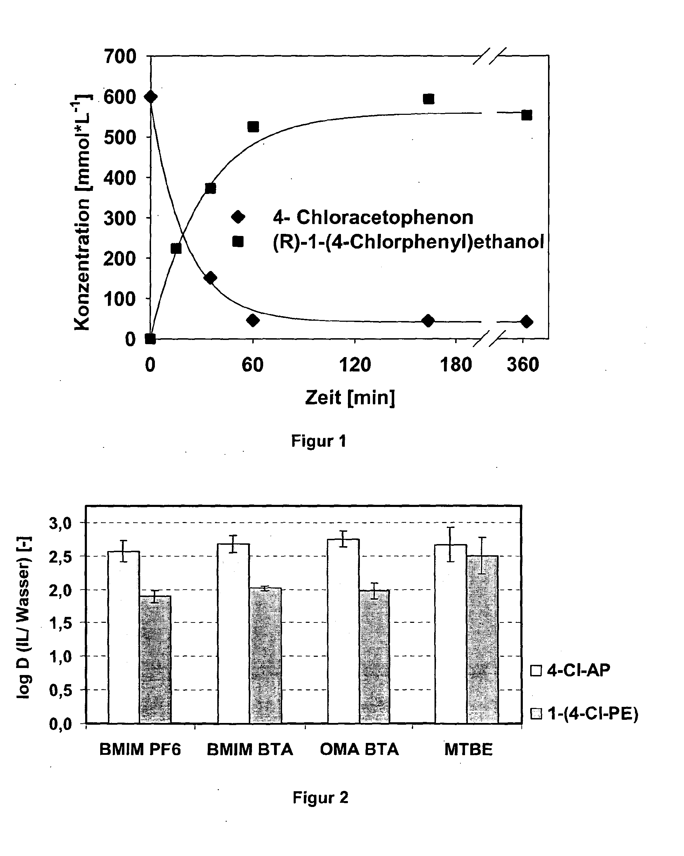

Im folgenden wird ein Beispiel für eine Prozessführung einer Ganzzellbiotransformation in zweiphasigen Systemen aus ionischer Flüssigkeit und Wasser unter Ausnutzung der zelleigenen Cofaktor-Regeneration beschrieben.
Im zweiphasigen System ionische Flüssigkeit/ Wasser bietet die wässrige Phase dem Biokatalysator optimale Bedingungen, die nicht mit Wasser mischbare ionische Flüssigkeit dient als Eduktreservoir und *in situ*-Extraktionsmittel. Als Reaktor kann dafür beispielsweise ein üblicher Rührkessel verwendet werden.
Je nach verwendeter ionischer Flüssigkeit können die Verteilungskoeffizienten von Substrat und Produkt so gewählt werden, dass keine Inhibierung des Biokatalysators durch diese Stoffe auftritt. Deren wässrige Konzentrationen liegen dann unterhalb der kritischen Konzentrationen. Sobald das Substrat von den Zellen umgesetzt wird, diffundiert neues Substrat aus der ionischen Flüssigkeit nach. Die Stofftransportrate kann dabei durch eine Vergrößerung der Stoffaustauschfläche mittels Erhöhung des Leistungseintrags über das Rührorgan gegebenenfalls gesteigert werden. Das entstehende Produkt gelangt wieder per Diffusion zurück in die ionische Flüssigkeit und reichert sich dort an. Am Ende der Reaktion kann die ionische Flüssigkeit leicht zum Beispiel mittels Sedimentation oder Zentrifugation von der Zellsuspension abgetrennt werden. Eine Produktabtrennung ist im folgenden unproblematisch durch Destillation, Extraktion, Membranfiltration oder andere Verfahren möglich.
Der Anteil der wässrigen Phase kann unter anderem davon abhängig gemacht werden, wie viel Wasser die Zellen für eine optimale Umgebung benötigen und wie viel für eine ausreichende Versorgung mit Cosubstraten etc. wie zum Beispiel Glukose oder Sauerstoff gebraucht wird.
Ein Beispiel für das erfindungsgemäße Verfahren ist die asymmetrischen Reduktion von 4-Chloracetophenon zu (R)-1-(4-Chlorphenyl)ethanol mit lebenden Zellen von *Lactobacillus kefir* in 20 % ionischer Flüssigkeit ("BMIM BTA": 1,3-Butyl-Methylimidazolium Bis-Trifluormethansulphonimide) im Satzverfahren.
In der gepufferten wässrigen Phase befindet sich neben den Zellen (50 g*L⁻¹) das Cosubstrat Glukose (0,2 mol*L⁻¹), das die Zellen zur Regeneration von zellinternem NADPH benötigen. Dabei wird Acetat als Nebenprodukt gebildet. In der ionischen Flüssigkeit wird das Substrat (0,6 mol*L⁻¹ 4-Chloracetophenon) vorgelegt. Die Mischung wird von dem Rührorgan im Reaktor dispergiert. Nach ca. 2 Stunden ist die Reaktion beendet. In der ionischen Flüssigkeit findet sich ca. 0,54 mol*L⁻¹ (R)-1-(4-Chlorphenyl)ethanol mit einer exzellenten Reinheit von >99,5 % ee (Enantiomerenüberschuss). Dies entspricht in etwa einer Ausbeute von 90 % und einer Produktkonzentration von 85 g/*L⁻¹. Die Raumzeitausbeute ist mit fast 10 g*L⁻¹*h⁻¹ außerordentlich. Figur 1 zeigt den Verlauf einer solchen Reaktion.

### Beispiel 2:

### Auswahl der ionischen Flüssigkeit

Die Auswahl von BMIM BTA erfolgte nach einer Untersuchung der Lösungsmitteleigenschaften und der Biokompatibilität und anschließender Überprüfung der Eignung für die Ganzzellbiotransformation.
Die guten Lösungsmitteleigenschaften der untersuchten nicht mit Wasser mischbaren ionischen Flüssigkeiten 1,3-Butyl-Methylimidazolium Hexafluorophosphat (BMIM PF₆), BMIM BTA und Methyl-Trioctylammonium Bis-Trifluormethansulphonimide (OMA BTA) zeigt sich in der kompletten Mischbarkeit der Lösungsmittel mit 4-Chloracetophenon und (R)-1-(4-Chlorphenyl)ethanol sowie deren Verteilungkoeffizienten zwischen den ionischen Flüssigkeiten und Wasser (Figur 2). Diese sind mit log D ~ 2,7 für 4-Chloracetophenon gleich wie für das gute organische Lösungsmittel Methyl-tertButylether (MTBE). Für 1-(4-Chlorphenyl)ethanol liegen sie mit log D - 2,0 etwas niedriger als der Wert für MTBE (log D ~ 2,5). Damit befinden sich aber bei Verwendung der ionischen Flüssigkeiten immer noch etwa 99 % des Produktes in der ionischen Flüssigkeit (im Gleichgewicht).

### Beispiel 3:

### Analyse der Membranintegrität der Zellen

In Figur 3 ist die Membranintegrität der Zellen nach einer fünfstündigen Inkubation unter Reaktionsbedingungen im Vergleich zum rein wässrigen Ansatz dargestellt. Über die Membranintegrität wird häufig die Viabilität abgeschätzt, da die Membran die lebenswichtige Barrierefunktion erfüllt. Gemessen wurde die Integrität mit dem Viabilitätstest "Life/Dead" von Molecular Probes, der die differenzierte passive Aufnahme von Fluoreszenzfarbstoffen in die Zellen quantitativ ermittelt. Man erkennt, dass beim Einsatz von BMIM BTA die Membranintegrität nur leicht vermindert ist, wohingegen bei den beiden anderen eine Abnahme auf circa 30 % zu beobachten ist. Beim rein wässrigen Ansatz erkennt man eine komplette Schädigung der Zellen durch das Substrat und/oder das Produkt.

### Beispiel 4:

### Eignung der ionischen Flüssigkeiten für die Ganzzellbiotransformation

Die Eignung der ionischen Flüssigkeiten für die Ganzzellbiotransformation wurde anhand der Reduktion von 4-Chloracetophenon mit *Lactobacillus kefir* untersucht. Figur 4 zeigt, dass alle drei untersuchten ionischen Flüssigkeiten zu sehr guten Ergebnissen führen.

### Beispiel 5:

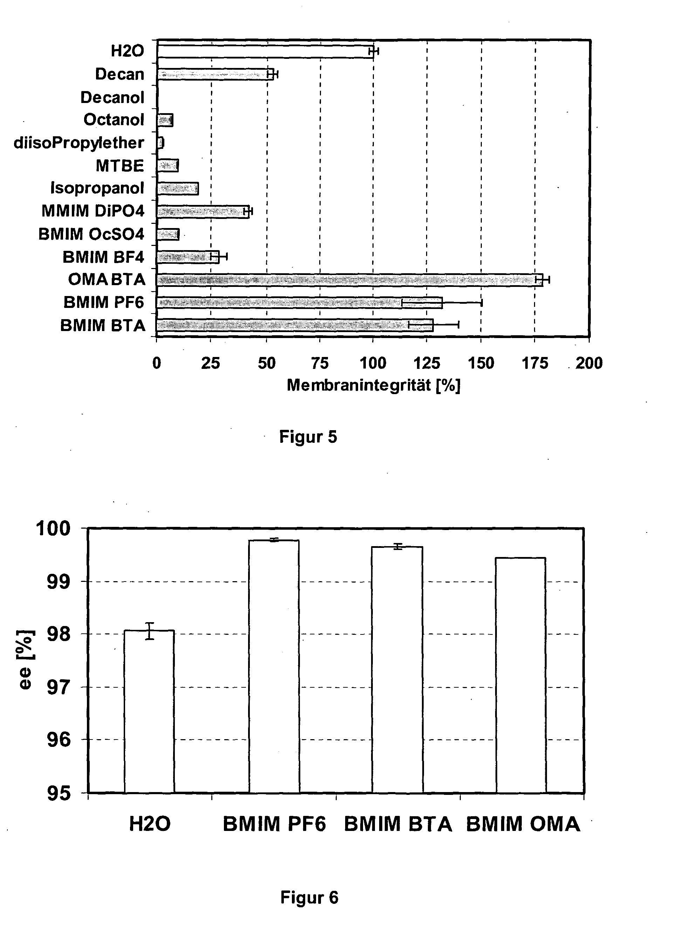

Durch das erfindungsgemäße Verfahren können überraschenderweise effizientere Prozesse zur Ganzzell-katalysierten Herstellung von Feinchemikalien entwickelt werden. Neben einer Steigerung der Ausbeute, der Produktkonzentration und der Raumzeitausbeute kann auch die Produktreinheit erhöht werden.
Die Verwendung von nicht mit Wasser mischbaren ionischen Flüssigkeiten nutzt die Vorteile eines Zweiphasensystems, die niedrige Konzentration von potenziell zellschädigenden Substraten und Produkten in der Katalysatorphase und die hohen Konzentrationen dieser Stoffe in der Lösungsmittelphase, die für eine gute Prozessausbeute nötig sind. Durch die zellschonenden Eigenschaften der ionischen Flüssigkeiten können diese Vorteile in vollem Umfang genutzt werden. Figur 5 zeigt die Membranintegrität von *Lactobacillus kefir-Zellen* nach fünfstündige Inkubation in 20 %igen Mischungen von wässriger Phase und verschiedenen organischen Lösungsmitteln und ionischen Flüssigkeiten im Vergleich zum rein wässrigen Ansatz.
Es konnte gezeigt werden, dass organische Lösungsmittel sehr schädlich für die Zellmembran sind, vermutlich aufgrund ihrer Fähigkeit, sich in die Membran einzulagern. Decan, das mit 5,6 einen hohen log P-Wert besitzt, zeigt eine geringere Zellschädigung, wie aus vergleichbaren Literaturdaten zu erwarten war (Inoue A und Horikoshi K, 1991). Es ist jedoch als Lösungsmittel aufgrund des unpolaren Charakters schlecht geeignet. Die mit Wasser mischbaren ionischen Flüssigkeiten 1,3-Dimethylimidazolium Diphosphat (MMIM DiPO₄), 1,3-Butyl-Methylimidazolium Octylsulphat (BMIM-OcSO₄) und 1,3-Butyl-Methylimidazolium Tetrafluoroborat (BMIM BF₄) führen ebenso zu einer mehr oder weniger ausgeprägten Zellschädigung.
Die nicht mit Wasser mischbaren ionischen Flüssigkeiten BMIM PF₆, BMIM BTA und OMA BTA zeigen keine Beeinträchtigung der Membranintegrität, es ist im Gegenteil sogar eine Verstärkung der Barrierefunktion zu beobachten. Wie in Figur 3 dargestellt, wird diese unter Reaktionsbedingungen durch das Substrat und das Produkt wieder aufgehoben, sodass es nicht zu einer im Vergleich zum rein wässrigen Ansatz erhöhten Stofftransportlimitierung kommt (siehe Figur 4).
Einer der überraschesten Vorteile, der sich aus der guten Biokompatibilität der ionischen Flüssigkeiten ergibt, ist die Möglichkeit der Nutzung der Regenerationssysteme des Biokatalysators. Bei Vorlage des Cosubstrats in der wässrigen Phase kann der Zusatz teurer Cofaktoren vermieden werden, da die intakten Zellen diesen immer wieder regenerieren können und aufgrund der intakten Membran nicht verlieren. Dies bedeutet unendliche Zykluszahlen (Menge des gebildeten Produkts pro Menge zugesetzten Cofaktors).
Es konnte gezeigt werden, dass durch die Verwendung von nicht mit Wasser mischbaren ionischen Flüssigkeiten eine sehr hohe Produktreinheit erreicht werden kann. Der Enantiomerenüberschuss von (R)-1-(4-Chlorphenyl)ethanol wurde im zweiphasigen System ionische Flüssigkeit/ Wasser im Vergleich zum rein wässrigen Ansatz von 98,0 % auf über 99,5 % gesteigert (siehe Figur 6).
Durch die räumliche Trennung des Biokatalysators und des Produkts in zwei verschiedenen Phasen sowie die Vermeidung von Zellaggregationen, die bei Verwendung von organischen Lösungsmitteln oft beobachtet wird (Cull et al. 2000), wird die Produktaufbereitung erleichtert. Die Lösungsmittelphase kann durch einfache Sedimentation oder Zentrifugation gewonnen werden. Das Produkt kann daraus durch Destillation, Extraktion, Pervaporation (WO 03/013685) Membranfiltration (WO 03/039719) oder ein anderes Standardverfahren gewonnen werden. Außerdem können die Zellen für einen neuen Ansatz wieder verwendet werden.
Die ionische Flüssigkeit kann nach der Produktabtrennung ebenfalls wieder verwendet werden. Im Gegensatz zur Arbeit mit organischen Lösungsmitteln ist dies durch den extrem geringen Dampfdruck verlustfrei möglich. Gegebenenfalls kann die ionische Flüssigkeit auch leicht gereinigt werden.
Ferner stellt die gefahrlose Handhabung ionischer Flüssigkeiten gegenüber organischen Lösungsmitteln einen großen Vorteil dar. Die Arbeitssicherheit wird erhöht, die Umweltbelastung reduziert.

### Literatur

Cull SG et al. Biotechnol Bioeng **2000**, 69, 227.
EuropaBio White Biotechnology: Gateway to a more Sustainable Future; **2003**
Hage A et al. Tetrahedron Asymm **2001**, 12, 1025.
Himeda Y et al. J Mol Cat A: Chem **2003,** 195, 95.
Howarth J et al. Tetrahedron Lett **2001,** 42, 7517.
Hummel W et al. Adv Syn Cat **2003,** 345, 153.
Inoue A, Horikoshi K **1991,** J Ferm Bioeng, 71(3), 194-196
Kataoka M et al. Appl Micron Biotechnol **2003,** 62 (5-6): 437-445, 437.
Krishna SH Biotechnol Adv **2002,** 20, 239.
Laane C et al. Biotechnol Bioeng **1987,** 30, 81.
Matsuda T et al. Chem Comm **2003**, 10, 1198.
Nakamura K, et al. Tetrahedron-Asymmetry **2003**, 14 (18): 2659-2681, 2659.
Ohkuma T et al. J Am Chem Soc **2002**, 124, 6508.
Rautenstrauch V et al. Chemistry-A European Journal **2003**, 9, 4954.
Seelbach K et al. Tetrahedron Letters **1996**, 37, 1377.
US Patent 4,925,790 ; Blanch HW et al. **1990;**
Villela M et al. Angew. Chem. Ang Chem Int Ed **2003,** 42, 2993.
WO 02/038784; Kragl U et al. 2002;
WO 03/013685; Goulao Crespo und Schäfer **2003**;
WO 03/039719; Wasserschied et al. 2003;
Wills M und Hannedouche J Curr Opin Drug Di De **2002**, 5, 881.

## Patentansprüche

1. Verfahren zur Herstellung von einem oder mehreren Produkten in einer Reaktion aus einem oder mehreren Edukten in Gegenwart von lebenden Bakterien oder eukaryontischen Zellen als Biokatalysatoren unter Verwendung eines mindestens zwei flüssige Phasen umfassenden Systems, umfassend eine erste biokompatible Phase einer ionischen Flüssigkeit und eine zweite Phase einer wässrigen Flüssigkeit, wobei die ionische Flüssigkeit und die wässrige Flüssigkeit heterogen zueinander sind und im Verfahren
(a) die erste Phase als Eduktreservoir dient; und
(b) die zweite Phase die lebenden Bakterien oder eukaryontischer Zellen enthält;
wobei die Phasen intensiv miteinander in Kontakt gebracht werden, das eine oder die mehreren Edukte durch Stofftransportvorgänge in die zweite Phase eintreten, dort durch die Biokatalysatoren in das eine oder die mehreren Produkte umgesetzt werden und das eine oder die mehreren Produkte nach der Umsetzung aus dem zweiphasigen System abgetrennt werden.

2. Verfahren nach Anspruch 1, wobei die Reaktion unter Ausnutzung der bakerieneigenen/zelleigenen Regeneration von verbrauchten Reaktionspartnern abläuft.

3. Verfahren nach Anspruch 1 oder 2, wobei die ionische Flüssigkeit der ersten Phase in einem Temperaturbereich von 0°C bis 90°C flüssig ist.

4. Verfahren nach einem der Ansprüche 1 bis 3, wobei das eine oder die mehreren Produkte durch Stofftransportvorgänge in die erste Phase übertreten und diese erst Phase zusätzlich als in-situ-Extraktionsmittel dient.

5. Verfahren nach einem der Ansprüche 1 bis 4, wobei der Stofftransportvorgang, durch den das eine oder die mehreren Edukte in die zweite Phase eintreten und/oder das eine oder die mehreren Produkte in die erste Phase eintreten, ausgewählt ist aus der Gruppe bestehend aus konvektivem Stofftransport, diffusivem Stofftransport und Stoffdurchgang an der Phasengrenze.

6. Verfahren nach einem der Ansprüche 1 bis 5, wobei die erste Phase ausgewählt ist aus Hexafluorophosphatsalzen (PF₆⁻) und Bis-Trifluormethansulfonamidsalzen (BTA⁻).

7. Verfahren nach einem der Ansprüche 1 bis 6, wobei die zweite Phase eine wässrige synthetische Nährlösung für die lebenden Bakterien oder eukaryontischer Zellen ist.

8. Verfahren nach einem der Ansprüche 1 bis 7, wobei das Verfahren in einem Reaktor ausgeführt wird, der ein Rührkessel ist.

9. Verfahren nach einem der Ansprüche 1 bis 8, wobei das Verfahren ein Satz-Verfahren ist, bei dem das eine oder die mehreren Produkte nach Abschluss der Umsetzung aus dem zweiphasigen System abgetrennt wird/werden.

10. Verfahren nach einem der Ansprüche 1 bis 9, wobei die Temperatur der zweiten Phase während der Umsetzung in einem Bereich von von 0°C bis 90°C liegt.

11. Verfahren nach einem der Ansprüche 1 bis 10, wobei der pH-Wert in der zweiten Phase während der Umsetzung in einem Bereich von 2 bis 10 liegt.

12. Verfahren nach einem der Ansprüche 1 bis 11, wobei die Edukte in der wässrigen Phase toxisch für die Bakterien oder eukaryontischen Zellen sind.

13. Zusammensetzung umfassend ein zweiphasiges System aus einer ersten Phase einer biokompatiblen ionischen Flüssigkeit und einer zweiten Phase einer wässrigen Flüssigkeit, wobei die ionische Flüssigkeit und die wässrige Flüssigkeit heterogen zueinander sind, wobei
(a) die erste Phase Edukte für eine Umsetzung zu Produkten durch einen Biokatalysator enthält; und
(b) die zweite Phase die lebenden Bakterien oder eukaryontischer Zellen als Biokatalysator enthält.
